# EUROPEAN PATENT APPLICATION

(11) **EP 2 532 350 A1**
(43) Date of publication of application: **12.12.2012**
(21) Application number: 12183545.8
(22) Date of filing: 13.10.2008
(51) Int. Cl.: A61K 31/00, A61K 31/03, A61K 31/56, A61K 45/06, A61P 43/00, A61K 31/444

(54) **Pharmaceutical combination of a glucocorticoid receptor antagonist and a cortisol synthesis inhibitor for treating cushing's syndrome**

(30) Priority: 17.10.2007 US 960856 P
(62) Divisional of application: 08805247.7
(71) Applicant: Laboratoire HRA Pharma, 75003 Paris (FR)
(72) Inventor: Ulmann, André, 75005 PARIS (FR); Gainer, Erin, 75019 PARIS (FR); Vuillet, François, 75017 PARIS (FR)
(74) Representative: Chajmowicz, Marion

(57) **Abstract**

The invention relates to a method for treating Cushing's syndrome in a patient, which method comprises administering the patient with a pharmaceutical composition comprising a glucocorticoid receptor antagonist, at least twice a day, or an extended-release composition of a glucocorticoid receptor antagonist, or a combination of a glucocorticoid receptor antagonist and a inhibitor of cortisol synthesis.

## Description

The invention relates to a method for treating Cushing's syndrome using glucocorticoid receptor antagonists.

### BACKGROUND OF THE INVENTION

Cushing's syndrome of endogenous origin is a hormonal disease with an estimated incidence of approximately 10 per 1 million persons (Meier and Biller, 1997). Cushing's syndrome is associated with an increased blood concentration of cortisol (hypercortisolism) or the presence in blood of glucocorticoid hormone excess over a long period of time. Cushing's syndrome is classified as either ACTH dependent or non ACTH dependent.
ACTH dependent Cushing's syndrome is characterised by a chronic ACTH hypersecretion which stimulates the growth of the adrenal glands and the hypersecretion of corticosteroids. The most common underlying cause of ACTH dependent Cushing's syndrome is excessive production of ACTH by pituitary adenomas known as Cushing's disease. Cushing's syndrome resulting from the production of ACTH in another location than the pituitary gland is known as ectopic Cushing's syndrome. Examples of ectopic sites include thymoma, medullary carcinoma of the thyroid, pheochromocytoma, islet cell tumours of the pancreas and small cell carcinoma of the lung.
ACTH independent Cushing's syndromes are caused by adrenal tumors that can be either adenomas or carcinomas. Both adrenal adenomas and carcinomas are characterised by chronic cortisol hypersecretion.
Symptoms of Cushing's syndrome include a characteristic abnormal fat deposition around the neck, thinning of the skin, osteoporosis, moon face, weakness, fatigue, backache, headache, impotence, muscle atrophy, increased thirst, urination, insulin resistance, dyslipidemia, myopathy, amenorrhea, hypertension, weight gain, central obesity, steroid hypersecretion, elevated urinary cortisol excretion and mental status changes, in particular depression (Orth 1995; Dahia and Grossman, 1999).

Effective drug therapies for Cushing's syndrome currently are not satisfactory. The oral inhibitors of adrenal steroidogenesis are the most commonly used medical agents in the treatment of Cushing's syndrome: these include metyrapone, ketoconazole, aminoglutethimide, mitotane and trilostane.
In ectopic ACTH secretion, when the tumor cannot be found or removed, medical therapy may be used to reduce cortisol production (Doppman et al, 1987, Doppman et al, 1989, Pass et al , 1990, Wajchenberg et al, 1994, Newell-Price et al, 1998). Furthermore, clinical trials showed some efficacy using high-dose mifepristone once a day (Nieman et al, 1985; Chrousos et al, 1989; van der Lely, 1991, Newfield et al, 2000; Chu et al, 2001). A fractioned dosage of mifepristone was successfully given to a young child (Beaufrère et al, 1987).

However in a long term, such high dosage of mifepristone given with long intervals between doses (e.g. once a day) triggers a massive secretion of cortisol due to interruption of the endogenous feedback mechanism. This high level of cortisol then overwhelms the blockage of the glucocorticoid receptors, leading to hypercortisolism (Raux-Demay et al, 1990).

### SUMMARY OF THE INVENTION

In order to avoid secretion of cortisol in response to the blockade of the glucocorticoid receptor, it is now proposed to give multiple low doses or a sustained-release low dosage of glucocorticoid receptor antagonist, and/or to combine the glucocorticoid receptor antagonist with an inhibitor of cortisol synthesis, for treating Cushing's syndrome.

The invention thus provides a method for treating Cushing's syndrome in an adult or an adolescent patient, which method comprises administering the patient with a pharmaceutical composition comprising a glucocorticoid receptor antagonist, at least twice a day.

The invention also relates to a glucocorticoid receptor antagonist for treating Cushing's syndrome in an adult or an adolescent patient by administration of said glucocorticoid receptor antagonist at least twice a day.

The invention also provides a method for treating Cushing's syndrome in a patient, which method comprises administering the patient with an extended-release composition of a glucocorticoid receptor antagonist.

The invention also relates to an extended-release composition of a glucocorticoid receptor antagonist for treating Cushing's syndrome in an adult or an adolescent patient.
The invention further provides a method for treating Cushing's syndrome in a patient, which method comprises administering the patient with a glucocorticoid receptor antagonist and an inhibitor of cortisol synthesis.

### DETAILED DESCRIPTION OF THE INVENTION

### Definitions:

Unless otherwise indicated, the patient to be treated may be any human subject afflicted with Cushing's syndrome, whatever the sex and the age of the subject. The patient may be a child, an adolescent (i.e. generally a subject who is 12 years old or above), or an adult. The patient to be treated is afflicted with Cushing's syndrome, preferably caused by ectopic ACTH secretion.

In the context of the invention, the glucocorticoid receptor antagonist may be a steroidal or non-steroidal glucocorticoid receptor antagonist.
Examples of steroidal glucocorticoid receptor antagonists include, without limitation, mifepristone, cortexolone, dexamethasone-oxetanone, 19-nordeoxycorticosterone, 19-norprogesterone, cortisol-21-mesylate; dexamethasone-21-mesylate, 11(-(4-dimethylaminoethoxyphenyl)-17(-propynyl-17(-hydroxy-4,9-estradien-3one, and 17(-hydroxy-17(-19-(4-methylphenyl)androsta-4,9(11)-dien-3-one.

In another preferred embodiment the steroidal glucocorticoid receptor is ulipristal, formerly known as CDB-2914, is 17α-acetoxy-11β-[4-*N*, *N*-dimethylamino-phenyl)-19-norpregna- 4, 9-diene-3, 20-dione, represented below.
It is a well-known steroid, more specifically a 19-norprogesterone, which possesses antiprogestational and antiglucocorticoidal activity. This compound, and methods for its preparation, are described in U. S. Patent Nos. 4,954, 490,5, 073,548, and 5,929, 262, and international patent applications WO2004/065405 and WO2004/078709, all incorporated herein by reference.

Other steroidal glucocorticoid receptor antagonists include metabolites of CDB-2914, as described in Attardi et al, 2004 , e.g. monodemethylated CDB-2914 (CDB-3877) ;didemethylated CDB-2914 (CDB-3963) ; 17alpha-hydroxy CDB-2914 (CDB-3236) ; aromatic A-ring derivative of CDB-2914 (CDB-4183).
Still other steroidal glucocorticoid receptor antagonists include metabolites of mifepristone, as described in Attardi et al, 2004, e.g. monodemethylated mifepristone, didemethylated mifepristone, and 17-α-[3'-hydroxy-propynyl] mifepristone.
These steroidal glucocorticoid receptor antagonists, as well as other derivatives, are represented below.

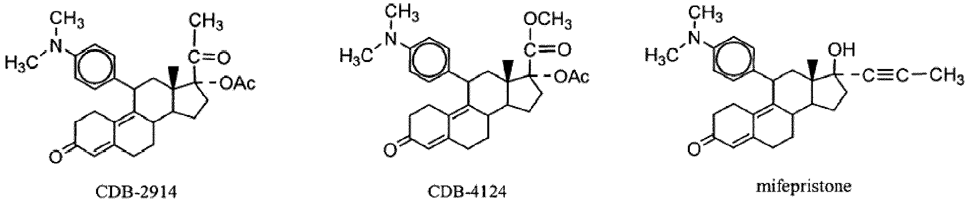

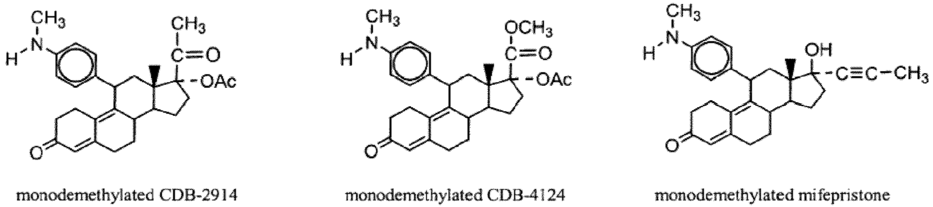

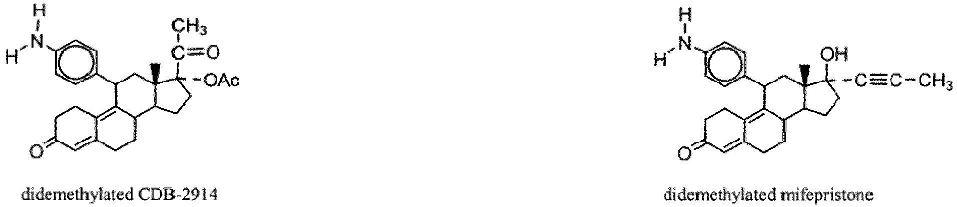

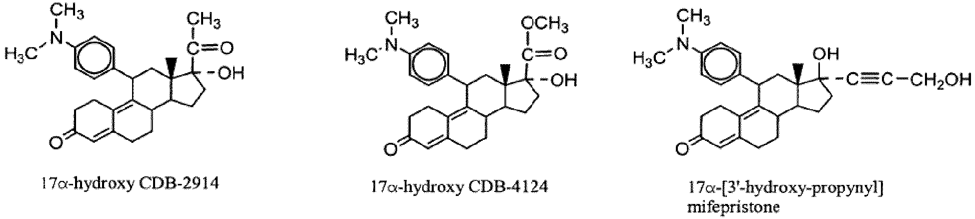

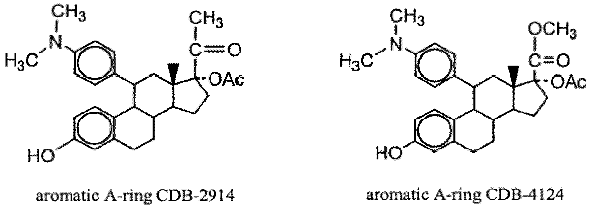

In a most preferred embodiment, the steroidal glucocorticoid receptor antagonist is mifepristone.

Examples of non-steroidal glucocorticoid receptor antagonists include, without limitation, N-(2-[4,4',441 -trichlorotrityl]oxyethyl)morpholine; 1-(2[4,4',4"-trichlorotrityl]oxyethyl)-4-(2-hydroxyethyl)piperazine dimaleate; N-([4,4',4"]-trichlorotrityl)imidazole; 9-(3-mercapto- 1,2,4-triazolyl)-9-phenyl-2,7-difluorofluorenone; 1-(2-chlorotrityl)-3,5-dimethylpyrazole; 4-(morpholinomethyl)-A-(2-pyridyl)benzhydrol; 5-(5-methoxy-2-(N-methylcarbamoyl)-phenyl)dibenzosuberol; N-(2-chlorotrityl)-L-prolinol acetate; 1-(2-chlorotrityl)-1,2,4-triazole; 1,S-bis(4,4', 4"-trichlorotrityl)-1,2,4-triazole-3-thiol;, 4.alpha.(S)-Benzyl-2(R)-chloroethynyl-1,2,3,4,4.alpha.,9,10,10.alpha. (R)-octahydro-phenanthrene-2,7-diol ("CP 394531") , 4.alpha. (S)-Benzyl-2(R)-prop-l-ynyl-1,2,3,4,4.alpha.,9,10,10.alpha. (R)-octahydro-phenanthrene-2,7-diol ("CP-409069"), trans-(1 R,2R)-3,4-dichloro-N-methyl-N-[2-1 pyrrolidinyl)cyclohexyl]benzeneacetamide, bremazocine, ethylketocyclazocine and naloxone.

In another embodiment, the non-steroidal glucocorticoid antagonist is one of the series synthesized by Corcept therapeutics. WO2006/014394, incorporated herein by reference, reports the synthesis and biological characterization of 48 novel 5,6-substituted pyrimidine-2,4-dione GR modulators. The most active compounds are compounds of formula I 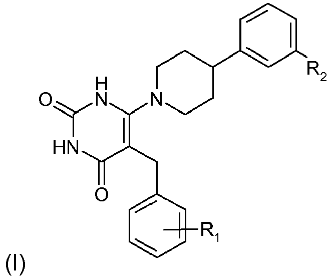 wherein
R1 is H and R2 is H or Cl,
or R1 is o-chloro or m-chloro and R2 is H.
In WO05/087769, incorporated herein by reference, Corcept therapeutics described the synthesis and biological testing of 150 compounds with a tetracyclic core ring structure that they term as azadecalins. Preferred azadecalin antagonists are compounds of formula II (II) 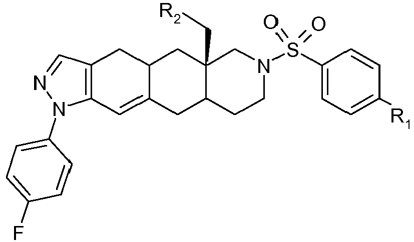 wherein
R1 is F and R2 is pyrrolidine,
or R1 is t-butyl and R2 is selected from the group consisting of H, a phenyl group, and - CH₂-O-CH₃

The compounds may be in the form of pharmaceutically acceptable salts, esters, optically active isomers, racemates or hydrates.

### Multiple doses

In a preferred embodiment, the invention provides a method for treating Cushing's syndrome in an adult or an adolescent patient, which method comprises administering the patient with a pharmaceutical composition comprising a glucocorticoid receptor antagonist, at least twice a day.
Most preferably, the patient is administered with pharmaceutical composition comprising a glucocorticoid receptor antagonist at least three times a day, e.g. three or four times a day.
Such chronic daily administration of the glucocorticoid receptor antagonist in subjects with Cushing's syndrome makes it possible to normalize glucocorticoid-dependent parameters through its cortisol-blocking action.
Preferably the daily dosage is less than about 40mg/kg/day, preferably less than about 20mg/kg/day.
The total daily amount of the glucocorticoid receptor antagonist administered may be advantageously inferior or equal to 800mg, preferably inferior or equal to 600mg, still preferably inferior or equal to 400mg, still more preferably inferior or equal to 300mg. The composition may be administered by any convenient route. The active ingredient may be administered by any convenient route, including oral, buccal, parenteral, transdermal, vaginal, uterine, rectal, nasal etc. Preferably the pharmaceutical composition is suitable for oral or parenteral administration. In a particular embodiment, the composition is in the form of an infusion to the patient.

### Extended-release form:

In another embodiment, the invention provides a method for treating Cushing's syndrome in a patient, which method comprises administering the patient with an extended-release composition of a glucocorticoid receptor antagonist.
By 'extended- release' is meant that the active ingredient is released from the formulation and thus made available for absorption by the body in a sustained manner, this being determined by the release rate controlling substance and interactions between the active ingredient, the release rate controlling substance and the media surrounding the formulation (e.g gastric juice).
Preferably the daily dosage is less than about 40mg/kg/day, preferably less than about 20mg/kg/day.
The total daily amount of the glucocorticoid receptor antagonist administered may be advantageously inferior or equal to 800mg, preferably inferior or equal to 600mg, still preferably inferior or equal to 400mg, still more preferably inferior or equal to 300mg. The composition may be administered by any convenient route. The active ingredient may be administered by any convenient route, including oral, buccal, parenteral, transdermal, vaginal, intra-uterine, rectal, nasal, etc. Preferably the pharmaceutical composition is suitable for oral or parenteral administration. In a particular embodiment, the extended-release composition is suitable for intradermal administration. For instance, the extended-release composition may be in the form of a patch or an implant. In still another embodiment, the extended-release composition is suitable for intra-uterine administration.

### Association with an inhibitor of cortisol synthesis:

The invention further provides a method for treating Cushing's syndrome in a patient, which method comprises administering the patient with a glucocorticoid receptor antagonist and an inhibitor of cortisol synthesis.
In a preferred embodiment, the inhibitor of cortisol synthesis is an adrenolytic agent. Preferably, the inhibitor of cortisol synthesis is mitotane. In another preferred embodiment, the inhibitor of cortisol synthesis is metyrapone.
Other examples of inhibitors of cortisol synthesis include, without limitation, aminoglutethimide, sodium valproate, an enkephalin, an opioid, clonidine, oxytocin, etomidate, trilostane, phenyltoin, procaine, vitamin C, a salicylate, cimetidine, and lidocaine, as well as ketoconazole, clotrimazole; N-(triphenylmethyl)imidazole; N-([2-fluoro-9-phenyl]fluorenyl) imidazole; and N-([2-pyridyl]diphenylmethyl)imidazole. Preferably the glucocorticoid receptor antagonist is mifepristone and the inhibitor of cortisol synthesis is mitotane, metyrapone, aminoglutethimide, fluconazole or ketoconazole.
Preferably the daily dosage is less than about 40mg/kg/day, preferably less than about 20mg/kg/day.
The total daily amount of the glucocorticoid receptor antagonist administered may be advantageously inferior or equal to 800mg, preferably inferior or equal to 600mg, still preferably inferior or equal to 400mg, still more preferably inferior or equal to 300mg. The composition may be administered by any convenient route. The active ingredients may be administered by any convenient route, including oral, buccal, parenteral, transdermal, vaginal, uterine, rectal, nasal, etc. Preferably the pharmaceutical composition is suitable for oral or parenteral administration. In a particular embodiment, the composition is in the form of an infusion to the patient.

### Routes of administration:

For a brief review of present methods for drug delivery, see, Langer, Science 249:1527-1533 (1990), which is incorporated herein by reference. Methods for preparing administrable compounds are known or are apparent to those skilled in the art and are described in more detail in, for example, Remington's Pharmaceutical Science, 17th ed., Mack Publishing Company, Easton, Pa. (1985), which is incorporated herein by reference, and which is hereinafter referred to as "Remington."

For solid compositions, conventional non toxic solid carriers may be used which include, for example, pharmaceutical grades of mannitol, lactose, starch, magnesium stearate, sodium saccharine, talcum, cellulose, glucose, sucrose, magnesium, carbonate, and the like. For oral administration, a pharmaceutically acceptable nontoxic composition is formed by incorporating any of the normally employed excipients, such as those carriers previously listed.

Oral solid dosage forms are preferentially compressed tablets or capsules. Compressed tablets may contain any of the excipients described above which are diluents to increase the bulk of the active ingredient so that production of a compressed tablet of practical size is possible. Binders, which are agents which impart cohesive qualities to powdered materials are also necessary. Starch, gelatine, sugars such as lactose or dextrose, and natural and synthetic gums are used. Disintegrants are necessary in the tablets to facilitate break-up of the tablet. Disintegrants include starches, clays, celluloses, algins, gums and crosslinked polymers. Lastly small amounts of materials known as lubricants and glidants are included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants. Procedures for the production and manufacture of compressed tablets are well known by those skilled in the art (See Remington).

Capsules are solid dosage forms using preferentially either a hard or soft gelatine shell as a container for the mixture of the active ingredient and inert ingredients. Procedures for production and manufacture of hard gelatin and soft elastic capsules are well known in the art (See Remington).

Buccal forms or devices are also useful, such as those described in U.S. patent application 20050208129, herein incorporated by reference. U.S. patent application 20050208129 describes a prolonged release bioadhesive mucosal therapeutic system containing at least one active principle, with an active principle dissolution test of more than 70% over 8 hours and to a method for its preparation. Said bioadhesive therapeutic system comprises quantities of natural proteins representing at least 50% by weight of active principle and at least 20% by weight of said tablet, between 10% and 20% of an hydrophilic polymer, and compression excipients, and comprising between 4% and 10% of an alkali metal alkylsulphate to reinforce the local availability of active principle and between 0.1 % and 1% of a monohydrate sugar.

For parenteral administration, fluid unit dosage forms are prepared utilizing the compounds and a sterile vehicle, water being preferred. The active ingredient, depending on the vehicle and concentration used, can be either suspended or dissolved in the vehicle. In preparing solutions the compound can be dissolved in water for injection and filtered sterilized before filling into a suitable vial or ampoule and sealing. Advantageously, adjuvants such as a local anesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection is supplied to reconstitute the liquid prior to use. Parenteral suspensions can be prepared in substantially the same manner except that the compounds are suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the active ingredient.

Additionally, a suppository can be employed to deliver the active ingredient. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate. These suppositories can weigh from about 1 to 2.5 gm.

Transdermal delivery systems comprising a penetration enhancer and an occlusive backing are of use to deliver the active ingredient. Examples of penetration enhancers include dimethyl sulfoxide, dimethyl acetamide and dimethylformamide.

Systems comprising polymeric devices which slowly release or slowly erode and release within the body to provide continuous supplies of the active ingredient are also of use.

The below example illustrates the invention without limiting its scope.

### EXAMPLE:

### Case-study:

A 53 year-old female subject, first presented with clinical symptoms of Cushing's syndrome in August 2006, and she was diagnosed with Cushing's syndrome secondary to ectopic ACTH secretion in March 2007. She received 200mg mifepristone, three times a day (in the morning, at noon, and in the evening) for 2.5 weeks before dose reduction for 1 week to 400 mg (200 mg twice a day).

The administration of mifepristone rapidly improved (after 2 weeks of treatment) general clinical consequences of hypercortisolism: glycemia returned to normal, insulin was stopped and dose of metformin decreased by two. The dose of enalapril previously administered for hypertension was decreased from 30mg to 10 mg.

### References

- Attardi et al, Journal of Steroid Biochemistry & Molecular Biology, 2004, 88: 277-288
- Beaufrère et al, The Lancet, July 25, 1987, page 217
- Chrousos et al, "Clinical applications of RU486, a prototype glucocorticoid and progestin antagonist in : Adrenal and hypertension" Eds F. mantero, BA Scoggins, R. Takeda, EG Biglieri" JW Funder, Raven Press (NY), 1989, pp273-84
- Chu et al, 2001, J Clin Endocrinol Metab, 86:3568-73
- Dahia and Grossman, 1999, Endocr. Rev. 20:136-55
- Doppman et al, 1987, Radiology, 163:501-3
- Doppman et al, 1989, Radiology, 172:115-24
- Meier and Biller, 1997, Endocrinol Metab Clin North Am 26:741-762
- Newell-Price et al, 1998, Endocr Rev, 19:647-72
- Newfield et al, 2000; J Clin Endocrinol Metab, 2000, 85:14-21
- Nieman et al, 1985; J Clin Endocrinol Metab, 1985, 61:536-40
- Orth, 1995, N. Engl. J. Med. 332:791-803
- Pass et al , 1990, Ann Thorac Surg, 50 :52-7
- Raux-Demay et al, 1990; J Clin Endocrinol Metab, 1990,70 :230-33
- van der Lely, 1991, Ann Intern Med, 114:143-144
- Wajchenberg et al, 1994, Endocr Rev, 15:752-87

## Claims

1. A method for treating Cushing's syndrome in a patient, which method comprises administering the patient with a glucocorticoid receptor antagonist and a inhibitor of cortisol synthesis.

2. The method of claim 1, wherein the glucocorticoid receptor antagonist is a steroidal glucocorticoid receptor antagonist.

3. The method of claim 2, wherein the steroidal glucocorticoid receptor antagonist is selected from the group consisting of mifepristone, , monodemethylated mifepristone, didemethylated mifepristone, 17-α-[3'-hydroxy-propynyl] mifepristone, ulipristal, CDB-3877, CDB-3963, CDB-3236, CDB-4183, cortexolone, dexamethasone-oxetanone, 19-nordeoxycorticosterone, 19-norprogesterone, cortisol-21-mesylate; dexamethasone-21-mesylate, 11(-(4-dimethylaminoethoxyphenyl)-17(-propynyl-17(-hydroxy-4,9-estradien-3one, and 17(-hydroxy-17(-19-(4-methylphenyl)androsta-4,9(11)-dien-3-one.

4. The method of claim 3, wherein the steroidal glucocorticoid receptor antagonist is mifepristone.

5. The method of claim 1, wherein the glucocorticoid receptor antagonist is a non-steroidal glucocorticoid receptor antagonist.

6. The method of claim 5, wherein the non-steroidal glucocorticoid receptor antagonist is selected from the group consisting of N-(2-[4,4',441 - trichlorotrityl]oxyethyl)morpholine; 1-(2[4,4',4"-trichlorotrityl]oxyethyl)-4-(2-hydroxyethyl)piperazine dimaleate; N-([4,4',4"]-trichlorotrityl)imidazole; 9-(3-mercapto- 1,2,4-triazolyl)-9-phenyl-2,7-difluorofluorenone; 1-(2-chlorotrityl)-3,5-dimethylpyrazole; 4-(morpholinomethyl)-A-(2-pyridyl)benzhydrol; 5-(5-methoxy-2-(N-methylcarbamoyl)-phenyl)dibenzosuberol; N-(2-chlorotrityl)-L-prolinol acetate; 1-(2-chlorotrityl)-1,2,4-triazole; 1,S-bis(4,4', 4"-trichlorotrityl)-1,2,4-triazole-3-thiol;, 4.alpha.(S)-Benzyl-2(R)-chloroethynyl-1,2,3,4,4.alpha.,9,10,10.alpha. (R)-octahydro-phenanthrene-2,7-diol ("CP 394531") , 4.alpha. (S)-Benzyl-2(R)-prop-1-ynyl-1,2,3,4,4.alpha.,9,10,10.alpha. (R)-octahydro-phenanthrene-2,7-diol ("CP-409069"), trans-(1 R,2R)-3,4-dichloro-N-methyl-N-[2-1 pyrrolidinyl)cyclohexyl]benzeneacetamide, bremazocine, ethylketocyclazocine, naloxone and compounds of formula I 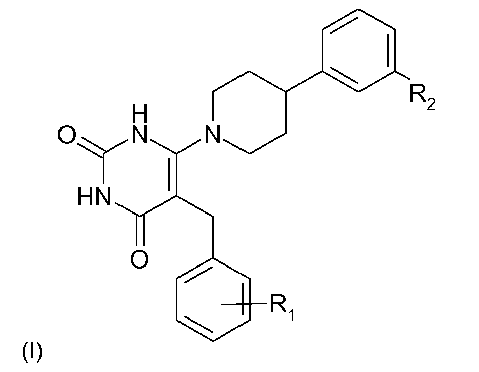 wherein
R1 is H and R2 is H or Cl,
or R1 is o-chloro or m-chloro and R2 is H. or compounds of formula II 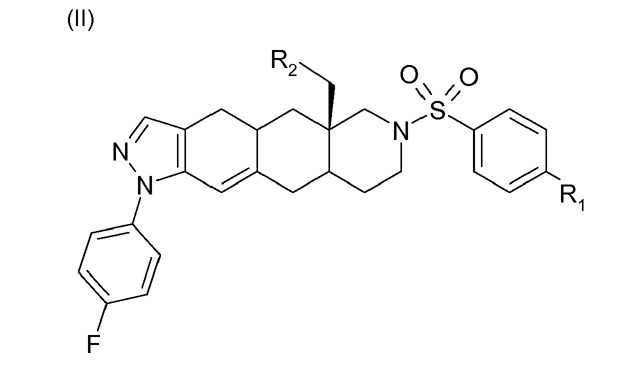 wherein
R1 is F and R2 is pyrrolidine,
or R1 is t-butyl and R2 is selected from the group consisting of H, a phenyl group, and -CH₂-O-CH₃..

7. The method of claim 1, wherein the inhibitor of cortisol synthesis is an adrenolytic agent.

8. The method of claim 7, wherein the inhibitor of cortisol synthesis is mitotane.

9. The method of claim 1, wherein the inhibitor of cortisol synthesis is metyrapone.

10. The method of claim 1, wherein the inhibitor of cortisol synthesis is selected from the group consisting of aminoglutethimide, sodium valporate, an enkephalin, an opioid, clonidine, oxytocin, etomidate, trilostane, phenytoin, procaine, vitamin C, a salicylate, cimetidine, and lidocaine

11. The method of claim 1, wherein the inhibitor of cortisol synthesis is selected from the group consisting of ketoconazole, clotrimazole; N-(triphenylmethyl)imidazole; N-([2-fluoro-9-phenyl]fluorenyl)imidazole; and N-([2-pyridyl]diphenylmethyl)imidazole, whatever the pharmaceutical form is.
